# EUROPÄISCHE PATENTSCHRIFT

(11) **EP 0 466 030 B1**
(45) Veröffentlichungstag und Bekanntmachung des Hinweises auf die Patenterteilung: **11.10.1995**
(21) Anmeldenummer: 91111126.8
(22) Anmeldetag: 04.07.1991
(51) Int. Cl.: C07K 5/06, A61K 38/05, A23L 1/305

(54) **Neue N-Acyldipeptide und deren Verwendung**
N-acyl dipeptides and their use
Dipeptides N-acylés et leur emploi

(30) Priorität: 12.07.1990 DE 4022267; 16.04.1991 EP 91105999
(43) Veröffentlichungstag der Anmeldung: 15.01.1992
(73) Patentinhaber: DEGUSSA AG, 60311 Frankfurt (DE)
(72) Erfinder: Drauz, Karlheinz, Dr., W-6364 Freigericht (DE); Knaup, Günter, Dr., W-6454 Bruchköbel (DE); Groeger, Ulrich, Dr., W-8750 Aschaffenburg (DE)

(56) Entgegenhaltungen:
- EP-A- 0 050 196
- GB-A- 2 071 760
- JOURNAL OF THE AMERICAN CHEMICAL SOCIETY. Bd. 111, Nr. 17, 16. August 1989, GASTON, PA US Seiten 6813 - 6821 J K BLODGETT AND G M LOUDON 'direct cleavage versus transpeptidation in the autodecomposition of peptides containing 2, 4-diaminobutanoic acid (DABA) and 2,3-diaminopropanoic acid (DAPA) residues. Specific cleavage of DAPA-containing peptides'
- JOURNAL OF BIOLOGICAL CHEMISTRY Bd. 262, Nr. 24, 25. August 1987, BALTIMORE, MD US Seiten 11435 - 11445 K KOBAYASHI AND J A SMITH 'acyl-peptide hydrolase fron rat liver: characterization of enzyme reaction'
- HELVETICA CHIMICA ACTA Bd. 65, Nr. 6, 22. September 1982, BASEL CH Seiten 1707 - 1719 S A BIZZOZERO ET AL. 'serine-protease-assisted synthesis of peptide substrates for alpha-chymotrypsin'
- CHEMICAL ABSTRACTS, vol. 110, no. 5, 30. Januar 1989, Columbus, Ohio, US; abstract no. 39353g, P STEHLE 'adequate supply of short chain peptides- a prerequisite for their use in clinical nutrition' Seite 584 ;Spalte L ;
- CHEMICAL ABSTRACTS, vol. 115, no. 2, 20. Januar 1992, Columbus, Ohio, US; abstract no. 15621v, K KOSEGI ET AL. 'infusion solutions containing n-acylated dipeptides and reducing sugars' Seite 452 ;Spalte R ;
- Derwent Zusammenfassung 91-012375

## Beschreibung

Die Erfindung betrifft neue N-Acyldipeptide der Formel

R²-NH-CHR¹-CO-AS

gemäß Anspruch 1 und deren Verwendung in Mischungen bzw. Lösungen für die enterale oder parenterale Ernährung oder in Nährmedien für Zellkulturen.

Dipeptide werden als Quelle für bestimmte Aminosäuren in Mischungen bzw. Lösungen für die künstliche Ernährung verwendet, so z.B. in EP-B-0 087 750, DE-C-31 08 079, EP-A-0 182 356.
Der Einsatz solcher Dipeptide ist vor allem dann angebracht, wenn die zu verabreichenden freien Aminosäuren schlecht wasserlöslich sind, wie Cystin, Tyrosin, Trytophan, Valin, Leucin oder Isoleucin, oder aber in wäßriger Lösung nicht beständig sind, wie bei Glutamin oder Cystein.

Die EP-A-0 220 379 beschreibt außerdem die Verwendung von Dipeptiden des Glutamins als Bestandteil für Nährmedien für Zellkulturen.

In der GB-A-2071760 wird die Verwendung von bestimmten Di- und Tripeptiden in Lösungen für die künstliche Ernährung beschrieben.

Sowohl Infusionslösungen als auch Nährmedien für Zellkulturen müssen in keimfreier Form eingesetzt werden. Die einfachste und üblicherweise angewandte Möglichkeit zur Sterilisation ist das kurzzeitige Erhitzen der fertigen Lösung auf eine Temperatur um 120°C. Unter diesen Bedingungen, aber auch schon bei längerer Lagerung der Lösungen können die bekannten Dipeptide zu 2,5-Diketopiperazinen cyclokondensieren. Diese 2,5-Diketopiperazine weisen jedoch eine höhere Epimerisierungstendenz auf als die entsprechenden linearen Dipeptide (J. Am. Chem. Soc., Vol. 108 (1988), 7327-7332). Über die physiologischen Wirkungen der 2,5-Diketopiperazine liegen bislang nur unzureichende Untersuchungen vor, so daß Dipeptide als Quelle für Aminosäuren zu oben genanntem Zweck problematisch sind.

Es ist auch bekannt, in Infusionslösungen als Quelle für bestimmte Aminosäuren die entsprechenden N-Acetylderivate einzusetzen, die aber biologisch nur unvollständig verwertet werden. Die Spaltung sowohl von N-Acetylaminosäuren als auch von bestimmten Dipeptiden findet zum überwiegenden Teil in den Nieren statt (Metabolism, Vol. 35 (1986), 830-836; Z. Ernährungswiss. 21 (1982), 21-26). Dipeptide werden darüberhinaus auch schon im Blutplasma gespalten.

In der EP-A-0 267 179 wurde vorgeschlagen, als Ersatz für die thermisch nicht beständigen Di- und Tripeptide des Cysteins entsprechende N-Acylpeptide, z.B. Bis-(acetylglycyl)-L,L-cystin, in Nutrienszusammensetzungen einzusetzen. Untersuchungen an Ratten haben jedoch gezeigt, daß Bis-(acetylglycyl)-L,L-cystin bei der parenteralen Ernährung nicht als Cysteinquelle geeignet ist (Z. Ernährungswiss. 28 (1989), 32 - 35).

Es ist auch schon ein N-acyliertes Dipeptid Ac-Ala-Gln als Zwischenstufe einer Transpeptidierungsreaktion beschrieben worden (J. Am. Chem. Soc. 111, 1989, S. 6814).

Aufgabe der vorliegenden Erfindung ist die Bereitstellung von Aminosäuren in einer Form, die ausreichend in Wasser löslich ist, z.B. für Infusionen, sterilisationsstabil ist, insbesondere in Lösungen nicht zu unerwünschten Nebenreaktionen neigt und die geeignet ist, vom Körper verwertet zu werden.

Gelöst wird diese Aufgabe mit neuen N-Acyldipeptiden der Formel

R²-NH-CHR¹-CO-AS (I)

in der AS für den C-terminalen Rest einer der Aminosäuren Isoleucin, Tyrosin, Glutamin und Cystein steht, sofern R¹ Wasserstoff und R² eine Formylgruppe bedeuten,
oder AS für den C-terminalen Rest einer der Aminosäuren Valin, Isoleucin, Tyrosin, Glutamin und Cystein steht, sofern R¹ eine Methylgruppe und R² eine Formylgruppe bedeuten,
oder AS für den C-terminalen Rest einer der Aminosäuren Isoleucin, Glutamin oder Cystein steht, sofern R¹ Wasserstoff und R² eine Acetylgruppe bedeuten oder für Isoleucin oder Cystein steht, sofern R¹ eine Methylgruppe und R² eine Acetylgruppe bedeuten,
oder AS für den C-terminalen Rest einer der Aminosäuren Valin, Leucin, Isoleucin, Tyrosin, Glutamin und Cystein steht, sofern R¹ Wasserstoff oder eine Methylgruppe und R² einen Acylrest einer aliphatischen Mono- oder Dicarbonsäure mit 3 bis 6 C-Atomen bedeuten, ausgenommen Methacrylsäure und ausgenommen, wenn R¹ eine Methylgruppe und AS Isoleucin ist, 3-Methylbuttersäure,
und deren physiologisch vertraglichen carbonsauren Salze.

Es wurde gefunden, daß überraschenderweise mit diesen Verbindungen und aus bestimmten weiteren N-Acyldipeptiden, bei denen die C-terminale Aminosäure nicht Cystin ist, im lebenden Organismus sehrwohl die C-termiale Aminosäure abgespalten wird, so daß diese N-Acyldipeptide als Quelle für diese Aminosäure geeignet sind. Im Gegensatz zu den freien Dipeptiden werden diese N-Acyldipeptide zwar nicht schon im Blutplasma gespalten, aber in der Niere findet eine rasche Spaltung statt. Dies konnte durch in-vitro-Untersuchungen mit Schweinenierenhomogenisat eindrucksvoll belegt werden. Beispielsweise wird N-Acetyl-L-alanyl-L-tyrosin 10 mal und N-Butyryl-L-alanyl-L-tyrosin sogar 15 mal schneller als N-Acetyl-L-tyrosin gespalten.

Weiterhin wurde überraschend festgestellt, daß N-Acyldipeptide als Quelle für die C-terminalen Aminosäuren in Nährmedien für Zellkulturen dienen können. Ein Vergleichsexperiment mit L1210 Mäuse-Leukaemiezellen zeigte eindrucksvoll, daß bei Sublimentation von entweder L-Glutamin, L-Alanyl-L-glutamin oder N-Acetyl-L-alanyl-L-glutamin kein signifikanter Unterschied im Zellwachstum feststellbar ist. In allen drei Fällen wurde jedoch ein deutlich erhöhtes Wachstum im Vergleich zum glutaminfreien Medium beobachtet. Die Tatsache, daß auch das N-Acetyl-L-alanyl-L-glutamin von den Zellen sehr gut verwertet wird ist umso erstaunlicher, als das vergleichbare N-Acetyl-L-glutamin von Zellen nicht oder nur sehr schlecht genutzt wird.

Da diese N-Acyldipeptide im Gegensatz zu den freien Dipeptiden hinaus unter den üblichen Hitzesterilisationsbedingungen (5 bis 20 Minuten bei 121°C) außerordentlich beständig sind, können sie mit Vorteil als Quelle für die jeweilige C-terminale Aminosäure, die z.B. in Folge ihrer Schwerlöslichkeit oder ihrer Instabilität nicht direkt für Infusionslösungen und Nährmedien für Zellkulturen geeignet ist, dienen.

Bezüglich der Verwendung von N-Acetyldipeptiden als Quelle für die C-terminalen Aminosäuren in Mischungen bzw. Lösungen für die enterale oder parenterale Ernährung oder von Nährmedien für Zellkulturen bezieht sich die vorliegende Erfindung auch auf Dipeptide der Formel

R²-NH-CHR¹-CO-AS (I')

in der AS für den C-terminalen Rest einer der Aminosäuren Valin, Leucin, Isoleucin, Tyrosin, Tryptophan, Glutamin und Cystein und R¹ Wasserstoff oder eine Methylgruppe und R² eine Formyl- oder Actylgruppe oder einen Acylrest einer aliphatischen Mono- oder Dicarbonsäure mit 3 bis 6 C-Atomen bedeuten, und deren physiologisch verträglichen carbonsauren Salze.

Vorzugsweise ist R¹ Wasserstoff oder eine Methylgruppe. Unter enterale oder parenterale Ernährung fällt die diätische oder diätetische sowie die künstliche Ernährung, das heißt die Ernährung über Magensonde oder Infusion.

Die erfindungsgemäßen bzw. die erfindungsgemäß zu verwendenden N-Acyldipeptide können mit freier endständiger Carboxylgruppe oder in Form ihrer physiologisch verträglichen carbonsauren Salze vorliegen. Als Kationen in solchen Salzen kommen in Frage beispielsweise Alkalimetalle, Erdalkalimetalle, Kationen von basischen Aminosäuren oder Kationen von Aminen der Formel NR³R⁴R⁵, wobei R³, R⁴ und R⁵ gleich oder verschieden sind und jeweils Wasserstoff, einen C₂-C₆-Hydroxyalkylrest oder C₁-C₆-Alkylrest bedeuten, oder zwei der Reste R³, R⁴ und R⁵ zu einem heterocyclischen Ring geschlossen sind, der gegebenenfalls noch ein Sauerstoffatom oder ein weiteres Stickstoffatom enthält.

Bevorzugte Acylreste R² in den Formeln (I) und (I') sind der Formyl-, Acetyl-, Propionyl-, Butyryl-, Succinyl- oder Hydroxysuccinylrest.

Die erfindungsgemäßen bzw. die erfindungsgemäß zu verwendenden N-Acyldipeptide können auf verschiedene Weisen einfach hergestellt werden. Zum einen können entsprechende N-Acylglycine oder N-Acylalanine nach bekannten Verfahren der Peptidchemie, z.B. über Aktivester, gemischte Anhydride oder enzymatisch mit Aminosäuren oder C-terminal geschützten Aminosäuren gekoppelt werden (vgl. Houben-Weyl, Band 15, "Synthese von Peptiden", Georg Thieme Verlag, Stuttgart 1974).

Zum anderen können auch bereits vorliegende freie Glycyl- oder Alanyldipeptide nachträglich mit Acylhalogeniden oder -anhydriden acyliert werden.

Die Umsetzung mit Acylhalogeniden oder -anhydriden einer aliphatischen Carbonsäure mit 2 bis 6 C-Atomen erfolgt unter Schotten-Baumann-Bedingungen, vorzugsweise in Wasser. Wenn es die Löslichkeitseigenschaften der Dipeptide und/oder des Acylierungsreagenzes erfordern, können als Lösungsmittel auch Gemische aus Wasser und einem mit Wasser mischbaren organischen Lösungsmittel, z.B. einem niederen Alkohol, Aceton oder Tetrahydrofuran, verwendet werden. Als Base für die Acylierungsreaktion wird Natriumhydroxid bevorzugt. Gegebenenfalls können zur Acylierung auch direkt wäßrige, salzhaltige Peptidlösungen eingesetzt werden, wie sie bei manchen Peptidsynthesen, z.B. bei der N-Carbonsäureanhydridmethode, anfallen.

Die Herstellung der N-Formyldipeptide kann analog zur Herstellung von N-Formylaminosäuren durch Umsetzung der freien Dipeptide mit dem gemischten Anhydrid aus Ameisensäure und Essigsäure erfolgen (vgl. Houben-Weyl. Band 15/1 "Synthese von Peptiden", Georg Thieme Verlag, Stuttgart 1974, Seiten 164 ff.).

In allen Fällen können die fertigen N-Acyldipeptide durch Ansäuern mit einer Mineralsäure in Freiheit gesetzt werden, wobei die in Wasser schwerlöslicheren N-Acyldipeptide direkt ausfallen. In Wasser leichtlösliche N-Acyldipeptide können mit einem mit Wasser nicht mischbaren organischen Lösungsmittel extrahiert werden. In Wasser leicht lösliche N-Acyldipeptide können auch durch Behandeln mit einem stark sauren Ionenaustauscher in der H⁺-Form in Freiheit gesetzt werden, wobei man sie dann in Form einer wäßrigen Lösung erhält.

Die carbonsauren Salze der N-Acyldipeptide werden durch Lösen äquimolarer Mengen des N-Acyldipeptids und der entsprechenden Base in Wasser hergestellt. Durch Einengen der Lösung oder durch Ausfällen mit einem mit Wasser mischbaren organischen Lösungsmittel können sie in fester Form gewonnen werden, vorzugsweise werden sie jedoch direkt in Form der erhaltenen wäßrigen Lösungen weiterverwendet.

Die Erfindung wird durch die nachfolgenden Beispiele näher erläutert:

### Beispiel 1:

0,1 Mol L-Alanyl-L-tyrosin wurden in 50 ml 2N Natronlauge gelöst. Bei 0°C wurden 0,11 Mol Acetanhydrid in 18 ml Tetrahydrofuran und 27,5 ml 4N Natronlauge so zugetropft, daß der pH-Wert des Reaktionsgemisches stets bei etwa 11 lag. Nach Beendigung der Zugabe wurde 1 Stunde lang nachgerührt, dann mit konzentrierter Salzsäure auf pH 1 angesäuert und das Volumen der Lösung auf die Hälfte eingeengt. Das N-Acetyl-L-alanyl-L-tyrosin kristallisierte aus, wurde abfiltriert, mit Wasser gewaschen und getrocknet.
- Ausbeute:: 11,08 g (38 % der Theorie)
- Schmelzpunkt:: 206°C
- ¹H-NMR (DMSO-d₆):: 1,17(d,3H), 1,83(s,3H), 2,87(dd,2H), 4,30(m,2H), 6,63(d,2H), 6,98(d,2H), 7,94(d,1H), 7,96(d,2H), 9,19(br.s,1H), 12,60(br.s,1H)
Die Spaltung des N-Acetyl-L-alanyl-L-tyrosins durch Schweinenierenhomogenisat wurde wie folgt untersucht:
140 g schlachtfrische Schweineniere wurden in kleine Stücke geschnitten und mit 200 ml eiskalter physiologischer Kochsalzlösung homogenisiert. 1 ml des nach 30-minütiger Zentrifugation bei 10000 U/min erhaltenen Überstandes wurden mit 1 ml 0,1 M Tris/HCl (pH 7)-Puffer und 1 ml einer 30 mM Lösung des Substrates in 0,1 M Tris/HCl (pH7)-Puffer versetzt und bei 37°C inkubiert. Die zeitliche Abnahme der Substratkonzentration wurde durch HPLC verfolgt.

Die so bestimmte spezifische Aktivität des Nierenhomogenisats gegenüber N-Acetyl-L-alanyl-L-tyrosin betrug 420 nMol pro Minute und g Niere.

Gegenüber einer zum Vergleich in gleicher Weise untersuchten Probe N-Acetyl-L-tyrosin zeigte das Nierenhomogenisat eine spezifische Aktivität von 43 nMol pro Minute und g Niere.

### Beispiel 2:

Zu 0,1 Mol L-Alanyl-L-tyrosin in 50 ml 2N Natronlauge wurden 0,12 Mol Butyrylchlorid und 0,12 Mol 4N Natronlauge so zugetropft, daß der pH-Wert des Reaktionsgemisches stets zwischen 10 und 11 lag. Nach beendeter Zugabe wurde mit konzentrierter Salzsäure auf pH 1 angesäuert und mit Essigsäureethylester extrahiert. Der Extrakt wurde unter vermindertem Druck zur Trockne eingedampft. Die Ausbeute an N-Butyryl-L-alanyl-L-tyrosin betrug 18,37 g (47 % der Theorie).
- Schmelzpunkt:: 27 - 28°C
- ¹H-NMR (DMSO-d₆):: 0,83(t,3H), 1,17(d,3H), 1,50(m,2H), 2,08(t,2H), 2,80(m,1H), 2,92(m,1H), 4,34(m,2H), 6,65(d,2H), 6,99(d,2H), 7,87(m,2H), 9,18(br.s,1H), 12,50(br.s,1H)
Bei der im Beispiel 1 beschriebenen Untersuchung zeigte das Nierenhomogenisat gegenüber N-Butyryl-L-alanyl-L-tyrosin eine spezifische Aktivität von 660 nMol pro Minute und g Niere.

### Beispiel 4:

Es wurde verfahren wie im Beispiel 1 mit dem einzigen Unterschied, daß anstelle von L-Alanyl-L-tyrosin 0,1 Mol L-Alanyl-L-valin eingesetzt wurden.
Die Ausbeute an N-Acetyl-L-alanyl-L-valin betrug 17,72 g (77 % der Theorie).
- Schmelzpunkt:: 147°C
- 1^{H}-NMR (DMSO-d₆):: 0,94(d,6H), 1,28(d,3H), 1,92(s,3H), 2,16(m,1H) 4,23(dd,1H), 4,49(dq,1H), 7,96(d,1H), 8,11(d,1H), 12,65(br.s,1H)

### Beispiel 5:

0,1 Mol L-Alanyl-L-leucin wurden mit Acetanhydrid acyliert wie im Beispiel 1. Beim Ansäuern des Reaktionsgemisches auf pH 1 kristallisierte das N-Acetyl-L-alanyl-L-leucin sofort aus. Die Ausbeute betrug 17,8 g (73 % der Theorie).
- Schmelzpunkt:: 145°C
- ¹H-NMR (DMSO-d₆):: 0,84(d,3H), 0,90(d,3H), 1,18(d,3H), 1,62(m,2H), 1,61(m,1H), 1,82(s,3H), 4.20(m,1H), 4,31(m,1H), 7,97(d,1H), 8,00(d,1H), 12,48(br.s,1H)

### Beispiel 6:

Es wurde verfahren wie im Beispiel 1 mit dem einzigen Unterschied, daß anstelle von L-Alanyl-L-tyrosin 0,1 Mol L-Alanyl-L-isoleucin eingesetzt wurden. Die Ausbeute an N-Acetyl-L-alanyl-L-isoleucin betrug 19,44 g (80 % der Theorie).
- Schmelzpunkt:: 124°C
- ¹H-NMR (DMSO-d₆):: 0,87(d,3H), 0,87(t,3H), 1,18(d,3H, 1,18(m,1H), 1,41(m,1H), 1,79(m,1H), 1,82(s,3H), 4,18(dd,1H), 4,39(dq,1H), 7,88(d,1H), 8,00(d,1H), 12,54 (br.s,1H)

### Beispiel 7:

0,05 Mol L-Alanyl-L-tryptophan wurden in 25 ml 2N Natronlauge gelöst. Bei 0°C wurden 0,055 Mol Acetanhydrid in 9 ml Tetrahydrofuran und 13,7 ml 4N Natronlauge so zugetropft, daß der pH-Wert des Reaktionsgemisches stets bei etwa 11 lag. Nach Beendigung der Zugabe wurde 1 Stunde lang nachgerührt und dann mit konzentrierter Salzsäure auf pH 1 angesäuert. Dabei kristallisierte das N-Acetyl-L-alanyl-L-tryptophan aus. Es wurde abfiltriert, mit Wasser gewaschen und getrocknet. Die Ausbeute betrug 14,74 g (93 % der Theorie).
- Schmelzpunkt:: 214°C (unter Zersetzung)
- ¹H-NMR (DMSO-d₆):: 1,18(d,3H), 1,79(s,3H), 3,12(m,2H), 4,32(dq,1H), 4,46(m,1H), 7,00(m,2H), 7,16(s,1H), 7,33(d,1H), 7,51(d,1H), 7,95(d,1H, 7,99(d,1H), 10,87(br.s,1H), 12,62(br.s,1H)

### Beispiel 8:

Es wurde verfahren wie im Beispiel 1 mit dem einzigen Unterschied, daß anstelle von L-Alanyl-L-tyrosin 0,1 Mol Glycyl-L-valin eingesetzt wurden. Die Ausbeute an N-Acetyl-glycyl-L-valin betrug 10,69 g (50 % der Theorie).
- Schmelzpunkt:: 133°C
- ¹H-NMR (D₂O):: 0,82(d,3H), 0,87(d,3H), 1,95(s,3H), 2,08(m,1H), 3,86(s,2H), 4,18(d,1H)

### Beispiel 9:

Es wurde verfahren wie im Beispiel 1 mit dem einzigen Unterschied, daß anstelle von L-Alanyl-L-tyrosin 0,1 Mol Glycyl-L-leucin eingesetzt wurden. Die Ausbeute an N-Acetyl-glycyl-L-leucin betrug 3,33 g (15 % der Theorie).
- Schmelzpunkt:: 125°C
- 1^{H}-NMR (DMSO-d₆):: 0,79(d,3H), 0,84(d,3H), 1,47(m,2H), 1,56(m,1H), 1,80(s,3H), 3,66(d,2H), 4,19(m,1H), 8,02(m,2H), 12,50(br.s,1H)

### Beispiel 10:

Es wurde verfahren wie im Beispiel 1 mit dem einzigen Unterschied, daß anstelle von L-Alanyl-L-tyrosin 0,1 Mol Glycyl-L-tyrosin eingesetzt wurden. Die Ausbeute an N-Acetyl-glycyl-L-tyrosin betrug 20,2 g (72 % der Theorie).
- Schmelzpunkt:: 162°C
- ¹H-NMR (DMSO-d₆):: 1,83(s,3H), 2,78(dd,1H), 2,92(dd,1H), 3,68(m,2H), 4,37(m,1H), 6,67(d,2H), 6,99 (d,2H), 7,98(d,1H), 8,02(d,1H), 9,20(br.s,1H), 12,66(br.s,1H)

### Beispiel 11:

Es wurde verfahren wie im Beispiel 1 mit dem einzigen Unterschied, daß anstelle von L-Alanyl-L-tyrosin 0,1 Mol Glycyl-L-glutamin eingesetzt wurden. Die Ausbeute an N-Acetyl-glycyl-L-glutamin betrug 23,70 g (96 % der Theorie).
- Schmelzpunkt:: 193°C (unter Zersetzung)
- ¹H-NMR (D₂O):: 1,90(m,1H), 2,02 (s,3H), 2,10(m,1H), 2,27(t,2H), 3,81(s,2H), 4,32(dd,1H)

### Beispiel 12:

Zu einer Lösung von 0,1 Mol L-Alanyl-L-tyrosin in 200 ml Ameisensäure wurden unter Kühlung 70 ml Acetanhydrid so zugetropft, daß die Temperatur zwischen 5 und 15°C blieb. Es wurde 2 Stunden lang ohne Kühlung nachgerührt. Dann wurden 80 ml Eiswasser zugegeben. Das Reaktionsgemisch wurde zur Trockne eingedampft und der Rückstand aus Wasser umkristallisiert. Die Ausbeute an N-Formyl-L-alanyl-L-tyrosin betrug 17,46 g (62 % der Theorie).
- Schmelzpunkt:: 163°C
- 1^{H}-NMR (DMSO-d₆):: 1,17(d,3H), 2,86(m,2H), 4,46(m,2H), 6,63(d,2H), 6,99(d,2H), 7,96(s,1H), 8,08(d,1H), 8,18(d,1H)

### Beispiel 13:

Es wurde verfahren wie im Beispiel 12 mit dem einzigen Unterschied, daß anstelle von L-Alanyl-L-tyrosin 0,1 Mol L-Alanyl-L-glutamin eingesetzt wurden. Der nach dem Eindampfen verbliebene Rückstand wurde aus Wasser/Ethanol umkristallisiert. Die Ausbeute an N-Formyl-L-alanyl-L-glutamin betrug 9,32 g (38 % der Theorie).
- Schmelzpunkt:: 150°C
- 1^{H}-NMR (DMSO-d₆):: 1,21(d,3H), 1,79(m,1H), 1,96(m,1H), 2,13(m,2H), 4,12(m,1H), 4,41(dq,1H), 6,77 (br.s,1H), 7,27(br.s,1H), 8,22(d,2H), 12,30(br.s,1H)

### Beispiel 14:

Es wurde verfahren wie im Beispiel 12 mit dem einzigen Unterschied, daß anstelle von L-Alanyl-L-tyrosin 0,1 Mol Glycyl-L-tyrosin eingesetzt wurden. Der nach dem Eindampfen verbliebene Rückstand wurde aus Ethanol/Aceton umkristallisiert. Die Ausbeute an N-Formyl-glycyl-L-tyrosin betrug 5,50 g (21 % der Theorie).
- Schmelzpunkt:: 182°C
- ¹H-NMR (DMSO-d₆):: 2,83(m,2H), 3,77(m,2H), 4,37(m,1H), 6,64(d,2H), 6,98(d,2H), 8,03(s,1H), 8,13(m,2H)

### Beispiel 15:

Zu einer Lösung von 0,05 Mol Glycyl-L-glutamin in 100 ml Ameisensäure wurden unter Kühlung 35 ml Acetanhydrid so zugetropft, daß die Temperatur zwischen 5 und 15°C blieb. Es wurde 2 Stunden lang ohne Kühlung nachgerührt. Dann wurden 40 ml Eiswasser zugegeben. Das Reaktionsgemisch wurde zur Trockne eingedampft und der Rückstand aus Methanol/Isopropanol umkristallisiert. Die Ausbeute an N-Formyl-glycyl-L-glutamin betrug 6,57 g (29 % der Theorie).
- Schmelzpunkt:: 168°C (unter Zersetzung)
- ¹H-NMR (DMSO-d₆):: 1,79(m,1H), 1,98(m,1H), 2,10(m,2H), 3,79(m,2H), 4,18(m,1H), 6,77(br.s,1H), 7,28(br.s,1H), 8,05(s,1H), 8,20(m,2H)

### Beispiel 16:

0,1 Mol L-Alanyl-L-glutamin wurden in 50 ml 2N Natronlauge gelöst. Bei 0°C wurden 0,11 Mol Propionsäureanhydrid in 18 ml Tetrahydrofuran und 27,5 ml 4N Natronlauge so zugetropft, daß der pH-Wert des Reaktionsgemisches stets bei etwa 11 lag. Nach Beendigung der Zugabe wurde noch 1 Stunde lang nachgerührt, dann mit konzentrierter Salzsäure auf pH 1 angesäuert und das Volumen der Lösung auf die Hälfte eingeengt. Das N-Propionyl-L-alanyl-L-glutamin kristallisierte aus, wurde abfiltriert, mit Wasser gewaschen und getrocknet.
- Ausbeute:: 19,41 g (71 % der Theorie)
- Schmelzpunkt:: 78 - 82°C
- ¹H-NMR(D₂O):: 0,98(t,3H), 1,28(d,3H), 1,89(m,1H), 2,10(m,1H), 2,15(m,2H), 2,24(q,2H), 4,17(q,1H), 4,27(dd,1H)

### Beispiel 17:

Es wurde verfahren wie im Beispiel 16 mit dem einzigen Unterschied, daß anstelle von L-Alanyl-L-glutamin 0,1 Mol L-Alanyl-L-tyrosin eingesetzt wurden. Die Ausbeute an N-Propionyl-L-alanyl-L-tyrosin betrug 17,28 g (56 % der Theorie).
- Schmelzpunkt:: 64°C
- ¹H-NMR(DMSO-d₆):: 0,98(t,3H), 1,18(d,3H), 2,10(q,2H), 2,87(m,2H), 4,32(m,2H), 6,67(d,2H), 7,01(d,2H), 7,90(m,2H)

### Beispiel 18:

Es wurde verfahren wie im Beispiel 16 mit dem einzigen Unterschied, daß anstelle von L-Alanyl-L-glutamin 0,1 Mol Glycyl-L-glutamin eingesetzt wurden. Die Ausbeute an N-Propionyl-glycyl-L-glutamin betrug 21,49 g (83 % der Theorie).
- Schmelzpunkt:: 130°C
- ¹H-NMR(D₂O):: 1,12(t,3H), 2,02(m,1H), 2,24(m,1H), 2,37(m,4H), 3,95(s,2H), 4,41(m,1H)

### Beispiel 19:

Zu 0,05 Mol L-Alanyl-L-leucin in 25 ml 2N Natronlauge wurden 0,06 Mol Butyrylchlorid und 0,06 Mol 4N Natronlauge so zugetropft, daß der pH-Wert des Reaktionsgemisches stets zwischen 10 und 11 lag. Nach beendeter Zugabe wurde mit konzentrierter Salzsäure auf pH 1 angesäuert und mit Essigsäureethylester extrahiert. Der Extrakt wurde unter vermindertem Druck zur Trockne eingedampft. Die Ausbeute an N-Butyryl-L-alanyl-L-leucin betrug 7,07 g (50 % der Theorie).
- Schmelzpunkt:: 155°C
- ¹H-NMR(DMSO-d₆):: 0,90(m,9H), 1,18(d,3H), 1,50(m,4H), 1,62(m,1H), 2,09(t,2H), 4,23(m,1H), 4,37(m,1H), 7,92(d,1H), 7,99(d,1H), 12,50(br.s,1H)

### Beispiel 20:

Es wurde verfahren wie im Beispiel 19 mit dem einzigen Unterschied, daß anstelle von Butyrylchlorid 0,06 Mol Hexanoylchlorid eingesetzt wurden. Die Ausbeute an N-Hexanoyl-L-alanyl-L-leucin betrug 8,77 g (58 % der Theorie).
- Schmelzpunkt:: 112°C
- ¹H-NMR(DMSO-d₆):: 0,90(m,9H), 1,18(d,3H), 1,24(m,4H), 1,52(m,4H), 1,62(m,1H), 2,10(t,2H), 4,21(m,1H), 4,36(m,1H), 7,91(d,1H), 7,96(d,1H), 12,60(br.s,1H)

### Beispiel 21:

Zu einer Lösung von 10 mMol L-Alanyl-L-isoleucin, 10 mMol Natriumhydroxid und 25 mMol Natriumhydrogencarbonat in 60 ml Wasser wurden 20 mMol Bernsteinsäureanhydrid zugegeben. Das Reaktionsgemisch wurde 15 Stunden lang bei Raumtemperatur gerührt und dann mit konzentrierter Salzsäure auf pH 2 angesäuert. Das Reaktionsgemisch wurde unter vermindertem Druck zur Trockne eingedampft und der Rückstand mit 50 ml Isopropanol extrahiert. Aus dem Extrakt wurde durch Zugabe von Ether das N-Succinyl-L-alanyl-L-isoleucin ausgefällt.
- Ausbeute:: 1,32 g (43 % der Theorie)
- Schmelzpunkt:: 118 - 120°C
- ¹H-NMR(DMSO-d₆):: 0,88(m,6H), 1,16(m,1H), 1,18(d,3H), 1,38(m,1H), 1,68(m,1H), 2,42(m,4H), 4,38(m,1H), 7,85(d,1H), 8,02(d,1H), 12,20(br.s,1H)

### Beispiel 22:

Es wurde verfahren wie im Beispiel 21 mit dem einzigen Unterschied, daß anstelle von L-Alanyl-L-isoleucin 10 mMol L-Alanyl-L-glutamin eingesetzt wurden. Die Ausbeute an N-Succinyl-L-alanyl-L-glutamin betrug 2,27 g (76 % der Theorie).
- Schmelzpunkt:: 106 - 108°C
- ¹H-NMR(D₂O):: 1,19(d,3H), 1,80(m,1H), 1,98(m,1H), 2,17(m,2H), 2,42(m,4H), 4,13(m,2H)

### Beispiel 23:

Es wurde verfahren wie im Beispiel 21 mit dem einzigen Unterschied, daß anstelle von L-Alanyl-L-isoleucin 10mMol Glycyl-L-glutamin eingesetzt wurden. Die Ausbeute an N-Succinyl-glycyl-L-glutamin betrug 2,23 g (82 % der Theorie).
- Schmelzpunkt:: 98 - 100°C
- ¹H-NMR(DMSO-d₆):: 1,78(m,1H), 1,95(m,1H), 2,16(m,2H), 2,46(m,4H), 3,76(m,2H), 4,18(m,1H), 8,12(d,1H), 8,20(m,1H), 10,60(br.s,2H)

### Beispiel 24:

Zu einer Lösung von 8 mMol L-Alanyl-L-tyrosin, 8 mMol Natriumhydroxid und 20 mMol Natriumhydrogencarbonat in 50 ml Wasser wurden 16 mMol Bernsteinsäureanhydrid zugegeben. Das Reaktionsgemisch wurde 15 Stunden lang bei Raumtemperatur gerührt und dann mit konzentrierter Salzsäure auf pH 2 angesäuert. Das Reaktionsgemisch wurde unter vermindertem Druck zur Trockne eingedampft und der Rückstand mit 50 ml Isopropanol extrahiert. Das Isopropanol wurde unter vermindertem Druck abgedampft und es hinterblieben 1,32 g (50 % der Theorie) N-Succinyl-L-alanyl-L-tyrosin in Form eines zähen Öls.
- ¹H-NMR(D₂O):: 1,20(d,3H), 2,49(m,2H), 2,62(m,2H), 3,05(m,1H), 3,22(m,1H), 4,24(q,1H), 4,63(m,1H), 7,03(d,2H), 7,30(d,2H)

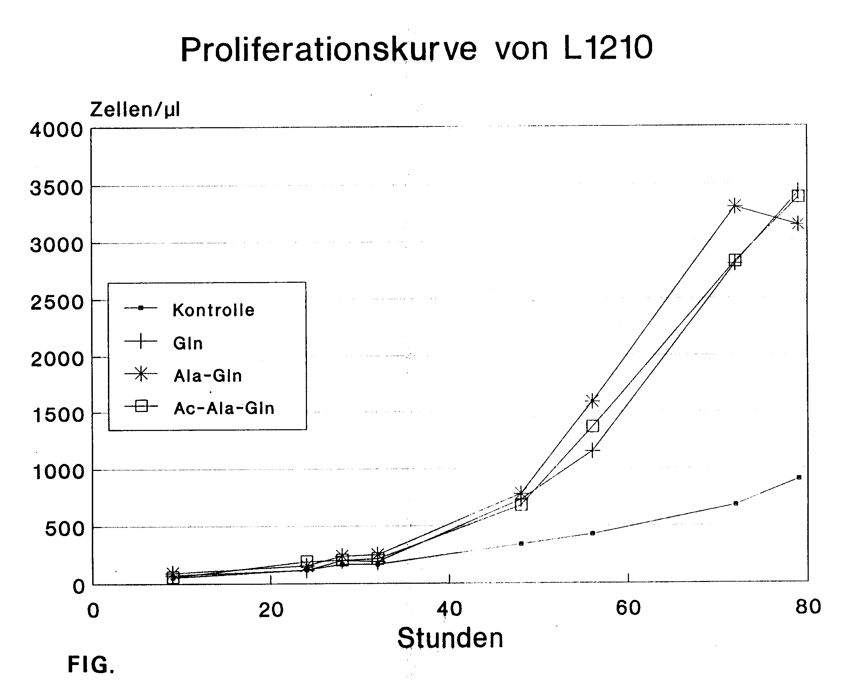

## Patentansprüche

1. N-Acyldipeptide der Formel
R²-NH-CHR¹-CO-AS (I)
in der AS für Isoleucin, Tyrosin, Glutamin oder Cystein steht, sofern R¹ Wasserstoff und R² eine Formylgruppe bedeuten,
oder AS für Valin, Isoleucin, Tyrosin, Glutamin oder Cystein steht, sofern R¹ eine Methylgruppe und R² eine Formylgruppe bedeuten,
oder AS für Isoleucin, Glutamin oder Cystein steht,
sofern R¹ Wasserstoff und R² eine Acetylgruppe bedeuten
oder AS für Isoleucin oder Cystein steht, sofern R¹ eine Methylgruppe und R² eine Acetylgruppe bedeuten,
oder AS für Valin, Leucin, Isoleucin, Tyrosin, Glutamin oder Cystein steht, sofern R¹ Wasserstoff oder eine Methylgruppe und R² einen Acylrest einer aliphatischen Mono- oder Dicarbonsäure mit 3 bis 6 C-Atomen bedeuten, ausgenommen Methacrylsäure und ausgenommen, wenn R¹ Methyl und AS Isoleucin ist, 3-Methylbuttersäure,
und deren physiologisch verträglichen carbonsauren Salze.

2. Verwendung von N-Acyldipeptiden der Formel
R²-NH-CHR¹-CO-AS (I')
in der AS für Valin, Leucin, Isoleucin, Tyrosin Tryptophan, Glutamin oder Cystein, R¹ Wasserstoff oder eine Methylgruppe und R² eine Formyl- oder Acetylgruppe oder einen Acylrest einer aliphatischen Mono- oder Dicarbonsäure mit 3 bis 6 C-Atomen bedeuten, und deren physiologisch verträglichen carbonsauren Salzen als Quelle für die C-terminalen Aminosäuren in Mischungen bzw. Lösungen für die enterale oder parenterale Ernährung oder von Nährmedien für Zellkulturen.

## Claims

1. N-acyl dipeptides of the formula
R² - NH - CHR¹ - CO - AS (1)
wherein AS represents isoleucine, tyrosine, glutamine or cysteine, provided that R¹ signifies hydrogen and R² signifies a formyl group,
or AS represents valine, isoleucine, tyrosine, glutamine or cysteine, provided that R¹ signifies a methyl group and R² signifies a formyl group,
or AS represents isoleucine, glutamine or cysteine, provided that R¹ signifies hydrogen and R² signifies an acetyl group,
or AS represents isoleucine or cysteine, provided that R¹ signifies a methyl group and R² signifies an acetyl group,
or AS represents valine, leucine, isoleucine, tyrosine, glutamine or cysteine, provided that R¹ signifies hydrogen or a methyl group and R² signifies an acyl radical of an aliphatic monocarboxylic acid or dicarboxylic acid having 3 to 6 C atoms, except methacrylic acid and, unless R¹ is methyl and AS is isoleucine, 3-methylbutyric acid,
and the physiologically compatible carboxylates thereof.

2. Use of N-acyl dipeptides of the formula
R² - NH - CHR¹ - CO - AS (1)
wherein AS represents valine, leucine, isoleucine, tyrosine, tryptophan, glutamine or cysteine, R¹ signifies hydrogen or a methyl group and R² signifies a formyl group or acetyl group or an acyl radical of an aliphatic monocarboxylic acid or dicarboxylic acid having 3 to 6 C atoms, and the physiologically compatible carboxylates thereof, as a source of C-terminal amino acids in mixtures and solutions for the enteral or parenteral nutrition of nutrient media for cell cultures.

## Revendications

1. N-acyldipeptides de formule :
R²-NH-CHR¹-CO-AS (I)
dans laquelle AS représente isoleucine, tyrosine, glutamine ou cystéine, dans la mesure où R¹ est l'hydrogène et R² un groupe formyle,
ou AS représente valine, isoleucine, tyrosine, glutamine ou cystéine, dans la mesure où R¹ représente un groupe méthyle et R² un groupe formyle,
ou AS représente isoleucine, glutamine ou cystéine, dans la mesure où R¹ représente l'hydrogène et R² un groupe acétyle, ou l'isoleucine ou la cystéine, dans la mesure où R¹ représente un groupe méthyle et R² un groupe acétyle,
ou AS représente valine, leucine, isoleucine, tyrosine, glutamine ou cystéine, dans la mesure où R¹ est l'hydrogène ou un groupe méthyle et R² un reste acyle d'un acide mono- ou dicarboxylique de 3 à 6 atomes de C, sauf l'acide méthacrylique et sauf quand R¹ est un groupe méthyle et AS est l'isoleucine, c'est l'acide 3-méthylbutyrique,
et leurs sels carboxyliques physiologiquement compatibles.

2. Utilisation de N-acyldipeptides de formule :
R²-NH-CHR¹-CO-AS (I')
dans laquelle AS représente valine, leucine, isoleucine, tyrosine, tryptophane, glutamine ou cystéine, R¹ est l'hydrogène ou un groupe méthyle et R² un groupe formyle ou acétyle ou un reste acyle d'un acide aliphatique mono- ou dicarboxylique de 3 à 6 atomes de C, et leurs sels d'acide carboxylique physiologiquement compatibles, comme source d'aminoacides C terminaux en mélanges ou en solution pour l'alimentation entérale ou parentérale ou pour des milieux de culture de cellules.
